(19) Europäisches Patentamt
European Patent Office
Office européen des brevets

(11) Publication number: **0 252 525 B1**

(12) **EUROPEAN PATENT SPECIFICATION**

(45) Date of publication of patent specification: **10.11.93**

(51) Int. Cl.5: **C07H 15/203**, C12Q 1/40, C12P 19/00, C12P 19/20

(21) Application number: **87110023.6**

(22) Date of filing: **10.07.87**

Divisional application 92116958.7 filed on 10/07/87.

# (54) Alpha-amylase assay using modified oligosaccharide and process for producing said modified oligosaccharide.

(30) Priority: **11.07.86 JP 163054/86**
**09.01.87 JP 2730/87**
**26.01.87 JP 15776/87**

(43) Date of publication of application:
**13.01.88 Bulletin 88/02**

(45) Publication of the grant of the patent:
**10.11.93 Bulletin 93/45**

(84) Designated Contracting States:
**AT BE CH DE ES FR GB GR IT LI LU NL SE**

(56) References cited:
**EP-A- 0 171 960**
**EP-A- 0 173 255**

**Chemical Abstracts, vol.103, no.1, July 8, 1985, page 256, abstract 2743p, Columbus, Ohio, US, K Omichi et al.: "Preparation of non-reducing-end substituted p-nitrophenyl alpha-maltopentaoside (FG5P) as a substrate for acoupled enzymic assay for alpha-amylases.**

(73) Proprietor: **WAKO PURE CHEMICAL INDUSTRIES LTD**
**1-2 Doshomachi-3-chome**
**Chuo-ku Osaka(JP)**

(72) Inventor: **Ikenaka, Tokuji**
**5-1, Midorigaokanakamachi-4-chome**
**Sakai-shi(JP)**
Inventor: **Omichi, Kaoru**
**31-10, Machikaneyamacho**
**Toyonaka-shi(JP)**
Inventor: **Satomura, Shinji**
**3-2-710, Nishimiyahara-3-chome**
**Yodogawa-ku**
**Osaka(JP)**
Inventor: **Natsuka, Yuko**
**1423, Natalia Lane No. 79**
**Ann Arbor, Michigan 48105(US)**

(74) Representative: **Baillie, Iain Cameron et al**
**c/o Ladas & Parry**
**Altheimer Eck 2**
**D-80331 München (DE)**

**EP 0 252 525 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for determining the activity of α-amylase in a sample using a modified oligosaccharide as a substrate and a process for producing such a modified oligosaccharide.

Heretofore, various precesses for determining the activity of α-amylase using a modified oligosaccharide as a substrate have been proposed. For example, U.S. Patent No. 4,649,108 to Blair discloses a method of measuring the amount of α-amylase in a liquid sample comprising the steps of providing an oligosaccharide substrate for α-amylase, said substrate containing at least 3 glucose units, the reducing-end glucose residue (unit) being bonded, via a bond cleavable by α- or β-glucosidase, to a label which exhibits an optically measurable change upon cleavage of said bond, and the non-reducing-end (terminal) glucose residue being bonded to a blocking group which inhibits cleavage by exo-enzymes of the bond between said non-reducing-end glucose residue and the adjacent glucose residue; contacting said sample with said oligosaccharide substrate and with a first added exo-enzyme capable of cleaving the bond between said reducing-end glucose residue and said label, and a second added exo-enzyme capable of cleaving bonds between glucose units, to form a mixture comprising said substrate, said first exo-enzyme, and said second exo-enzyme; and measuring said optically measurable change. According to said U.S. patent, the blocking groups can be carboxylic acid esters, phosphate esters, sulfonate esters, ethers (such as benzyl, silyl, and triphenylmethyl), monosaccharides other than α-1,4 linked glucose, and acetal or ketal blocking groups such as benzylidene (column 4 lines 37-67). But the ester blocking groups are unstable since they are easily hydrolyzed in an aqueous solution. Further, when the size of the blocking group becomes larger as in the case of toluenesulfonyl, methanesulfonyl, silyl or triphenylmethyl, the solubility in water is undesirably lowered. In addition, the introduction of a blocking group into the non-reducing-end glucose residue has many problems, and the ester and ether groups as mentioned above cannot be introduced into the non-reducing-end glucose residue by the process disclosed at from column 4 line 63 to column 7 line 58 of said U.S. patent. Therefore, according to said U.S. patent, preferably the substrate has eight or fewer glucose units, and most preferably has six or seven, and preferred blocking substituents are acetals or ketals, e.g. benzylidene (column 2 lines 4-7).

When the most preferred oligosaccharide according to said U.S. patent, that is, an oligosaccharide having 6 or 7 glucose units and benzylidene as the blocking group, is used as a substrate for measuring α-amylase, there are many problems in that the hydrolysis rate by α-amylase is slow, various kinds of hydrolyzed products are produced due to many positions to be hydrolyzed, the reaction for liberating the color producing group becomes many and complicated due to many hydrolyzed products, the amount of coupling enzymes to be used becomes larger, and thus the reliability for the measurement becomes insufficient. The improvement of these disadvantages has long been desired.

This invention provides a process for determining the activity of α-amylase in a sample which comprises

using as a substrate for α-amylase a modified oligosaccharide of the formula:

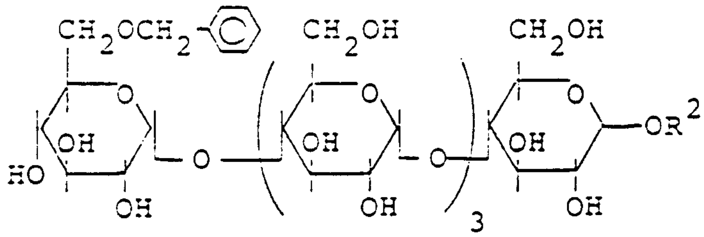

(I)

or

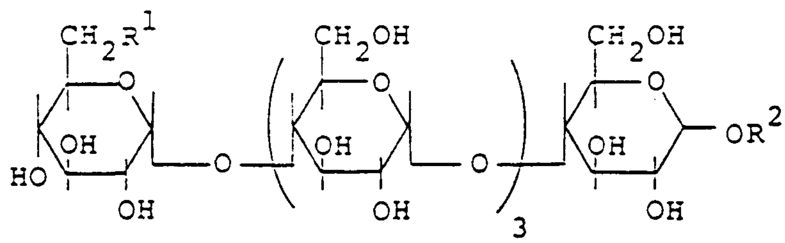

(II)

wherein $R^1$ is a halogen atom; and $R^2$ is a group of the formula:

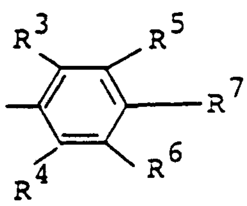 , 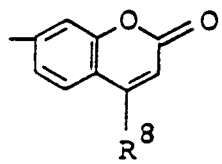 or 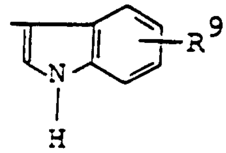

in which $R^3$ through $R^6$ are independently hydrogen, a lower alkyl group, a lower alkoxy group, a nitro group, a carboxyl group, a sulfonic acid group or a halogen atom, and $R^3$ and $R^5$, and/or $R^4$ and $R^6$, may be bonded to form an aromatic ring; $R^7$ is hydrogen, a lower alkoxy group, a halogen atom, or a nitro group; $R^8$ is hydrogen, a methyl group, or a trifluoromethyl group; and $R^9$ is hydrogen or a halogen atom,

contacting said sample with said substrate in the presence of at least one exo-enzyme, and

measuring an optically measurable change as a measure of α-amylase activity in said sample.

This invention also provides a process for producing an oligosaccharide represented by the formula (I) or (II), which comprises reacting a modified cyclodextrin with cyclomaltodextrin-gluconotransferase in the presence of an acceptor selected from glucose, maltose, and maltotriose wherein said glucose, maltose and maltotriose each carry a substituent linked to the sugar moiety by a glycosidic bond which upon cleavage exhibits an optically measurable change, and then reacting with glucoamylase or α-glucosidase.

This invention further provides a process for producing an oligosaccharide represented by the formula (I), which comprises reacting an oligosaccharide having a substituent which exhibits an optically measurable change upon cleavage at the reducing-end glucose residue with either benzaldehyde or benzaldehyde dimethylacetal to form the 4,6-0-cyclic acetal at the non-reducing-end glucose residue, and reducing said 4,6-0-cyclic acetal.

## BRIEF DESCRIPTION OF THE DRAWINGS

Fig. 1 is an IR chart of the oligosaccharide obtained in Example 1.

Figs. 2 and 3 are 'H-NMR charts of the oligosaccharides obtained in Examples 1 and 2, respectively.

Figs. 4 and 5 are calibration curves obtained in Examples 4 and 6, respectively.

The modified oligosaccharide used as a substrate for α-amylase is represented by the formula:

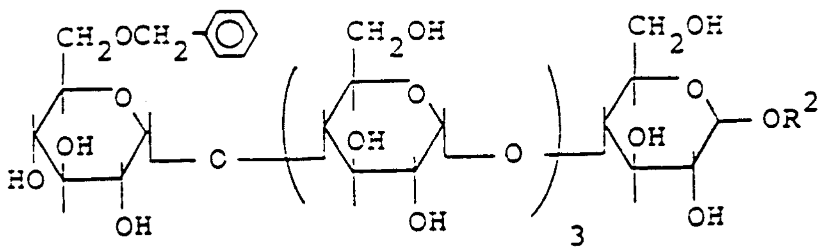

(I)

or

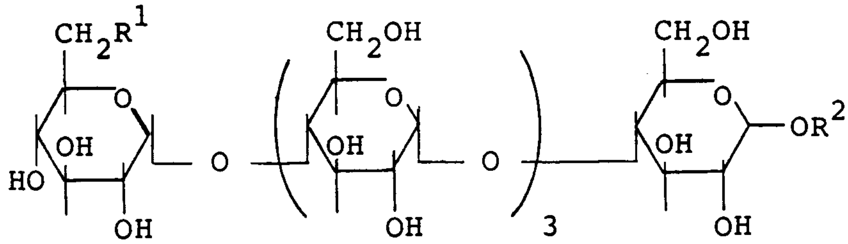

(II)

wherein $R^1$ is a halogen atom such as bromine, chlorine, iodine, etc.; and $R^2$ is a group of the formula:

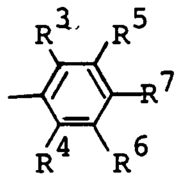, 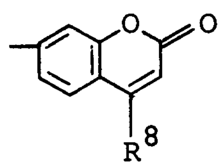 or 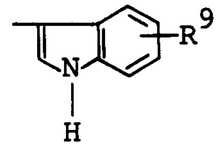

in which $R^3$ through $R^6$ are independently hydrogen, a lower alkyl group preferably having 1 to 4 carbon atoms, a lower alkoxy group preferably having 1 to 4 carbon atoms, a nitro group, a carboxyl group, a sulfonic acid group, or a halogen atom such as chlorine, bromine, iodine, etc., and $R^3$ and $R^5$, and/or $R^4$ and $R^6$, may be bonded to form an aromatic ring; $R^7$ is hydrogen, a lower alkoxy group preferably having 1 to 4 carbon atoms, a halogen atom such as chlorine, bromine, iodine, etc., or a nitro group; $R^8$ is hydrogen, a methyl group, or a trifluoromethyl group; and $R^9$ is hydrogen or a halogen atom such as chlorine, bromine, iodine, etc.

The oligosaccharides of the formula (I) and (II) have advantages in that the hydrolysis rate is fast, that is, the sensitivity as a substrate is high, the Michaelis constant ($K_m$) which is defined as a substrate concentration for giving a half of the maximum rate ($V_{max}$) of α-amylase is small, that is the specificity as a substrate is high and the required substrate concentration for measuring activities of α-amylase is low (usually used in a concentration of the value of $K_m$ x 5 to 10), and the position of hydrolysis is almost one position, that is, the color production reaction from the hydrolyzed products can be carried out simply and easily.

The above-mentioned advantages can be shown by using the following oligosaccharides:

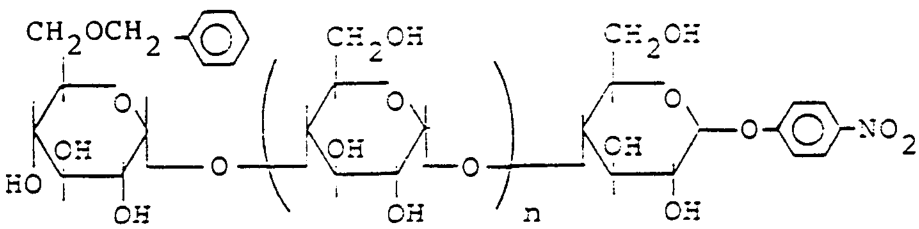 (III)

n = 2: BG4P
n = 3: BG5P (the present invention)
n = 4: BG6P
n = 5: BG7P

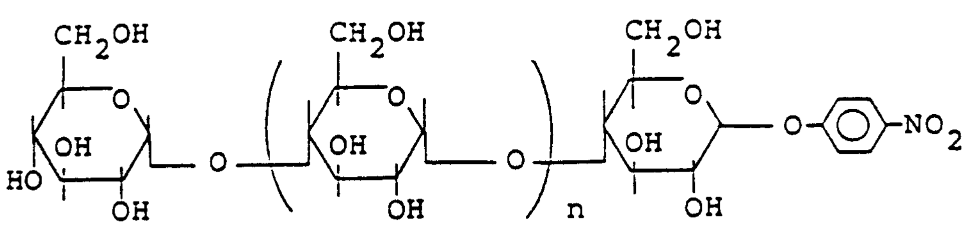

(IV)

n = 2: G4P
n = 3: G5P (standard)
n = 4: G6P
n = 5: G7P

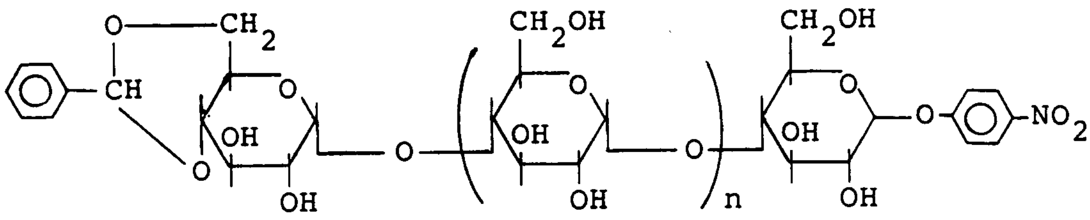

(V)

n = 2: BDG4P
n = 3: BDG5P
n = 4: BDG6P
n = 5: BDG7P

The hydrolysis rate is measured by using human pancreatic α-amylase (HPA) or human salivary α-amylase (HSA) for each substrate (1.0 mM) at pH 7, and evaluating by means of relative values by taking the value of G5P as 1.

The results are shown in Table 1.

Table 1

| Substrate | α-amylase | |
|---|---|---|
| | HPA | HSA |
| G4P | 0.70 | 0.75 |
| G5P | 1.0 | 1.0 |
| G6P | 1.7 | 1.7 |
| G7P | 1.8 | 1.8 |
| | | |
| BG4P | 0.55 | 0.60 |
| BG5P | 1.4 | 1.4 |
| BG6P | 1.4 | 1.4 |
| BG7P | 1.5 | 1.5 |
| | | |
| BDG4P | 0 | 0 |
| BDG5P | 0 | 0 |
| BDG6P | 0.4 | 0.5 |
| BDG7P | 1.1 | 1.0 |

The $K_m$ values were calculated by least square method according to a Lineweaver-Burle plot. The amounts of the products were measured with HPLC.

A mixture of 20 μℓ of α-amylase solution and 200 μℓ of 1.2 mM substrate solution in 50 mM BES [N,N-bis(2-hydroxyethyl)-2-aminoethanesulfonic acid]/NaOH buffer (pH 7.6), containing 20 mM sodium chloride and 2 mM calcium chloride was incubated at 37°C for 5 min. Then 25 μℓ of the reaction mixture was added to 500 μℓ of 5% acetic acid to stop the reaction. The sample (100 μℓ) was analyzed by HPLC to examine the amounts of the products.

The results are shown in Table 2.

Table 2

| Substrate | $K_m$ value | |
|---|---|---|
| | HPA | HSA |
| G5P | 0.36 mM | 0.31 mM |
| | 0.34 g/l | 0.29 g/l |
| BG5P | 0.11 mM | 0.10 mM |
| | 0.11 g/l | 0.10 g/l |
| BDG7P | 0.086 mM | 0.10 mM |
| | 0.12 g/l | 0.14 g/l |

The binding mode of each substrate were measured with high-performance liquid chromatography (HPLC) as described above.

The results are shown in Table 3.

Table 3

(%)

| α-amylase / Hydrolysis product / Substrate | HPA | | | | HSA | | | |
|---|---|---|---|---|---|---|---|---|
| | G4P | G3P | G2P | GP | G4P | G3P | G2P | GP |
| G4P | 0 | 0 | 55 | 45 | 0 | 0 | 74 | 26 |
| G5P | 0 | 12 | 73 | 15 | 0 | 11 | 83 | 6 |
| G6P | 0 | 76 | 22 | 2 | 0 | 53 | 40 | 7 |
| G7P | 55 | 30 | 14 | 1 | 60 | 30 | 8 | 2 |
| | | | | | | | | |
| BG4P | 0 | 0 | 8 | 92 | 0 | 0 | 10 | 90 |
| BG5P | 0 | 0 | 95 | 5 | 0 | 0 | 98 | 2 |
| BG6P | 0 | 73 | 23 | 4 | 0 | 68 | 29 | 3 |
| BG7P | 40 | 38 | 20 | 2 | 45 | 38 | 15 | 2 |
| | | | | | | | | |
| BDG4P<br>BDG5P | Not hydrolyzed | | | | Not hydrolyzed | | | |
| BDG6P | 0 | 12 | 73 | 15 | 0 | 10 | 66 | 24 |
| BDG7P | 13 | 22 | 48 | 7 | 15 | 25 | 55 | 5 |

As is clear from the results of Tables 1 to 3, only BG5P satisfies the three requirements, that is, the high hydrolysis rate, low $K_m$ value and almost one position at the hydrolysis in which G2P is produced mainly. Since G3P or G4P is not produced by the hydrolysis, it is possible to measure α-amylase activity by using as exo-enzyme only α-glucosidase which sufficiently hydrolyzes G2P but does not hydrolyze G3P or G4P sufficiently.

The activity of α-amylase can be measured by using the modified oligosaccharide of the formula (I) or (II) as follows:

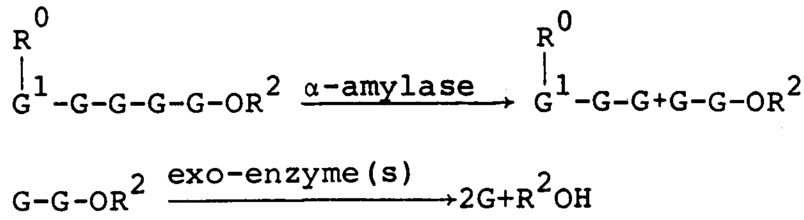


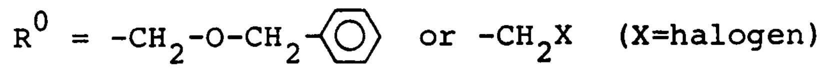

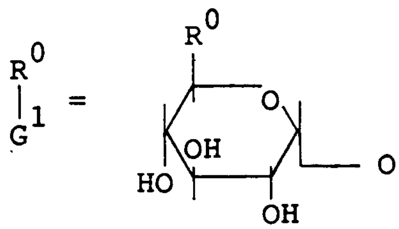

G: glucose residue

exo-enzyme(s):
(i) α-glucosidase
(ii) α-glucosidase, glucoamylase
(iii) α-glucosidase, β-glucosidase
(iv) α-glucosidase, β-glucosidase, glucoamylase
(v) isomaltase, α-glucosidase
(vi) isomaltase, glucoamylase

The substituent $R^0$ is attached to the $C_6$ position of the non-reducing-end glucose residue and the substituent $OR^2$ is attached to the $C_1$ position of the reducing end glucose residue and represented by, for example, a phenoxy group which may have one or more specified substituent groups, a naphthoxy group which may have one or more specified substituent groups, an umbelliferyl group with specified susbtituent groups, or an indoxyl group which may have one or more specified substituent groups.

That is the α-amylase in a sample first acts on the modified oligosaccharide of the formula (I) or (II) to produce

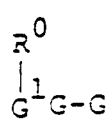

wherein the primary alcohol group ($-CH_2OH$) at the $C_6$ position of the non-reducing end glucose residue is replaced by the substituents $R^0$, and G-G-$OR^2$ wherein $OR^2$ is as defined above. Then, G-G-$OR^2$ is reacted with coupling enzyme(s) such as glucoamylase, α-glucosidase, β-glucosidase and the like to produce 2G and $R^2$-OH which exhibits an optically measurable change. When $R^2$-OH is a nitrophenol such as p-nitrophenol, the activity of α-amylase in a sample can be obtained by directly measuring the absorption spectrum thereof (e.g. absorbance at 405 nm). When $R^2$-OH is phenol or naphthol having no nitro group (or capable of having a nitro group) such as phenol, o-chlorophenol, 2,6-dichlorophenol, or p-methoxyphenol, it is reacted with a coupler such as 4-aminoantipyrine, or 3-methyl-2-benzothiazolinone hydrazone (MBTH) in the presence of an oxidase such as catechol oxidase, laccase, tyrosinase or monophenol oxidase, or an oxidant such as iodic acid or periodic acid; or peroxidase and hydrogen peroxide to produce a dye, the absorption spectrum of which is measured. When $R^2$-OH is a compound emitting fluorescence such as umbelliferone or 4-methylumbelliferone, the fluorescence intensity is measured. Further, when $R^2$-OH is indoxyl, the absorption spectrum of indigo dyes produced by oxidation is measured.

The concentration of the modified oligosaccharide used as a substrate in the determination of the activity of α-amylase is not particularly limited but usually is 0.1 to 10 mM.

As a sample to be measured, any ones containing α-amylase can be used. For example, there can be used as living body fluids blood, serum, urine, and saliva.

As the exo-enzyme which is a coupling enzyme, there can be used α-glucosidase, a mixture of α-glucosidase and glucoamylase, a mixture of α-glucosidase and β-glucosidase, a mixture of α-glucosidase, β-glucosidase and glucoamylase, a mixture of α-glucosidase and isomaltase, and a mixture of isomaltase and glucoamylase. These exo-enzymes may be derived from animals, plants and microorganisms. The amount of exo-enzymes used is usually 0.5 to 100 units/ml, preferably 2 to 50 units/ml.

The reaction temperature is not particularly limited and preferably 25° to 40°C. The reaction time can be selected properly depending on the purposes.

The pH of the reaction is not particularly limited and preferably 6 to 8. As a buffer for maintaining the correct pH there can be used a phosphate buffer, a tris(hydroxymethyl)aminomethane-HCl buffer, and a Good's buffer.

As an activation imparting agent for α-amylase, there can be used sodium chloride, calcium chloride, potassium chloride, and calcium acetate.

As the coupler for coupling (oxidation condensation) the phenol or naphthol liberated by the action of the exo-enzyme(s), there can be used 4-aminoantipyrine, 3-methyl-2-benzothiazolinonehydrazone (MBTH), and p-amino-N,N-diethylaniline.

As the oxidase for coupling (oxidation condensation) of the phenol or naphthol with the coupler, there can be used laccase, catechol oxidase, tyrosinase, and monophenol oxidase, derived from animals, plants, and microorganisms, in an amount of usually 0.2 to 10 units/ml, preferably 0.5 to 4 units/ml.

As the oxidizing agent for coupling (oxidation condensation), there can be used iodic acid and/or a salt thereof such as the sodium, and potassium salt, periodic acid and/or a salt thereof such as the sodium, potassium salt, and hydrogen peroxide.

Since the primary alcohol ($-CH_2OH$) at the $C_6$ position of the non-reducing-end glucose residue of the modified oligosaccharide of the formula (I) or (II) is replaced by the

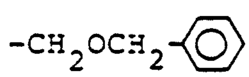

or $-CH_2X$ (X = halogen), the modified oligosaccharide of the formula (I) or (II) cannot be used as a substrate for glucoamylase, α-glucosidase, β-glucosidase or isomaltase as it is. But since the modified oligosaccharide of the formula (I) or (II) is easily soluble in water and excellent in affinity for α-amylase, it can be a good specific substrate for α-amylase. Thus, the α-amylase determination process using as a substrate the modified olicosaccharide of the formula (I) or (II) does not bring about side reactions and has a very small blank value. Further the reagent solution for the measurement is very stable. In addition, since human α-amylase almost only hydrolyses one glycosidic linkage of the modified oligosaccharide of formula (I) or (II) , the stoichiometry is established, which results in making the kinetic determination of α-amylase possible.

Moreover, in the process for determining the activity of α-amylase of this invention, since the main product produced by reacting with α-amylase is G2P, the duration of the lag period can be shortened by reacting with at least one coupling enzyme (exo-enzyme) such as glucoamylase, α-glucosidase, isomaltase or β-glucosidase in the reaction after the α-amylase reaction. Thus the determination of the α-amylase

activity can be carried out with high sensitivity and good linearity.

In the process for determining the activity of α-amylase of this invention, the measurement of an optically measurable change can be carried out by measuring absorption spectra of nitrophenols or indigo dyes liberated, by measuring absorption spectra of dyes formed by oxidation coupling of phenols or naphthols liberated with 4-aminoantipyrine, or MBTH, or by measuring fluorescence intensity of umbelliferone liberated, so that the determination process is hardly influenced by sugars such as glucose, and maltose, and reducing substances such as ascorbic acid, and bilirubin, present in samples to be measured.

The α-amylase activity determination process of this invention can be applied to either a rate assay wherein the reaction rate under certain conditions is measured, or an end point assay wherein a reaction terminator is used.

Further, the α-amylase activity determinating process of this invention is well suited to an auto-analyzer and if necessary can be applied in a manual method.

In addition, since a so-called colorimetric method (wherein the colour of a developed dye is measured) can be used by the use of the modified oligosaccharide of the formula (I) or (II), the process of this invention can also be applied to a test paper method which is a very simple method and a so-called dry determination process wherein multi-layer analysis sheets containing reaction reagents (multi-layer one-body type quantitative analysis films) are used.

The modified oligosaccharides of the formula (I) and (II) can be produced by reacting a modified cyclodextrin with cyclomaltodextrin-glucanotransferase in the presence of a specified acceptor, and then reacting with glucoamylase or α-glucosidase.

As the modified cyclodextrin, there is used those having one modified glucose reside per molecule.

As the modified glucose residue, there can be used substituted glucose wherein the primary hydroxyl group in the glucose is replaced by a substituent which is a benzyloxy group, or a halogen atom such as chlorine or bromine.

As the acceptor, there can be used glucose, maltose, or maltotriose having as a substituent, e.g. p-nitrophenyl, phenyl, umbelliferyl, naphtyl. When the glucose chain becomes longer, the glucose chain of major product in the initial reaction also becomes longer. Therefore, the glucose chain length of 1 to 3 is properly selected and used as the acceptor.

As the substituent of the hydroxyl group at the $C_1$ position of the modified glucose residue of the acceptor, there can be used the group represented by the formula: $-OR^2$ wherein $R^2$ is as defined above.

Concrete examples of the $-OR^2$ groups are a p-nitrophenoxy group, a m-nitrophenoxy group, an o-chlorophenoxy group, a p-chlorophenoxy group, a 2,6-dichlorophenoxy group, an o-methoxyphenoxy group, a p-methoxyphenoxy group, an o-methylphenoxy group, an o-carboxyphenoxy group, an o-sulfophenoxy group, a 1-naphthoxy group, a 2-sulfo-1-naphthoxy group, a 2-carboxy-1-naphthoxy group, an umbelliferyl group, a 4-methyl-umbelliferyl group, and an indoxyl group.

The cyclomaltodextrin-glucanotransferase (hereinafter referred to as "CGTase") is not particularly limited to its origin and source. There can be used those derived from Bacillus macerans, Bacillus megaterium, Klebsiella pneumonie, and alcaligenous.

The modified cyclodextrin can easily be obtained by using α-, β- or γ-cyclodextrin as a starting material and reacting with various reactants for introducing the desired modifying groups thereinto according to processes for producing various modified glucoses described in, for example, Method in Carbohydrate Chemistry I (1962) to V (1965), published by Academic Press. The selection of α-, β- or γ-cyclodextrin can be determined depending on the glucose chain length of the desired modified oligosaccharide derivative and its maximum yield.

The pH of the reaction of the modified cyclodextrin with CGTase in the presence of an acceptor changes slightly depending on the origin of CGTase, but usually is 6 to 8. Any buffering agent which does not inhibit the enzymatic reaction can be used for maintaining the suitable pH. Examples of the buffering agents are Good's buffer, ammonium acetate buffer, carbonate buffer, and phosphate buffer.

The modified cyclodextrin is used in a concentration of preferably 1 to 50 mmol/l and the accpetor is used in a concentration of 1 mmol/l or more to the solubility limit.

The acceptor is preferably used in an amount of 5 moles or more per mole of the modified cyclodextrin.

The CGTase is preferably used in an amount of 50 to 5000 U/ml. The reaction is preferably carried out at 20 to 50 °C. After the enzymatic reaction, CGTase is deactivated by heating, for example at 90 °C or higher for 10 minutes or more, or by changing the pH, for example pH of 4.0 or less.

The reaction using glucoamylase or α-glucosidase can preferably be carried out at the most suitable pH, for example pH 4 to 6, and using glucoamylase or α-glucosidase in an amount of preferably 5 to 100 U/ml.

Alternatively, the modified oligosaccharide of the formula (I) can be produced by reacting an oligosaccharide having a substituent which exhibits an optically measurable change upon cleavage at the reducing-end glucose residue with either benzaldehyde or benzaldehyde dimethylacetal to form the 4,6-0-cyclic acetal at the non-reducing end glucose residue, and reducing said 4,6-0-cyclic acetal.

First the oligosaccharide derivative is reacted with the specified aldehyde or acetal to form a cyclic acetal between the $C_4$ and $C_6$ positions of the non-reducing-end-glucose residue. The oligosaccharide derivative is available commercially or can be synthesized by a process described in, for example, Japanese Patent Unexamined Publication No. 54-51892.

The specified aldehyde or acetal or ketal is used in an amount of preferably 1 to 100 moles per mole of the oligosaccharide derivative.

The reaction is preferably carried out in the presence of a Lewis acid catalyst such as p-toluenesulfonic acid, or zinc chloride, in a suitable solvent such as N,N-dimethylformamide (DMF) at from room temperature to a reflux temperature for 0.5 to 12 hours.

The resulting intermediate having the cyclic acetal is usually subjected to modification of other hydroxyl groups by acylation, followed by a reduction step.

The acylation can be carried out by a conventional process, for example, in the presence of a base such as pyridine and using as an acylating agent such as acetic anhydride, acetylchloride, benzoylchloride, etc.

The acylation step is not essential but is preferable to enhance the solubility in a reaction solvent used in the next step (the reduction step).

The reduction step is usually carried out by using a reducing agent in the presence of an acid catalyst such as aluminum chloride, boron trifluoride, zinc chloride, p-toluenesulfonic acid, methanesulfonic acid, hydrogen chloride gas (or blown into ether) etc., in a suitable solvent (e.g. tetrahydrofuran (THF), etc.) usually at 0 - 50 °C for several tens of minutes to several hours.

As the reducing agent, there can be used sodium cyanoborohydride, lithium aluminumhydride, pyridineborane, dimethylamineborane, trimethylamineborane, t-butylamineborane, or diborane. The reudcing agent is preferably used in an amount of 1 to 1000 moles per mole of the oligosaccharide derivative.

The acid catalyst is preferably used in an amount of 0.5 to 5 moles per mole of the reducing agent.

When the acyl protection is conducted, deacyltion is conducted by a conventional process, for example, by treating with a 0.01 to 1.0 N sodium methoxide-methanol solution for periods up to several tens of hours at from room temperature to slightly elevated temperatures to easily give the modified oligosaccharide

Individual after-treatments after individual steps can be carried out by conventional processes. The final product can be purified by a conventional process such as column chromatography.

Since the α-1,4 bond of the oligosaccharide is subjected to acid hydrolysis under acidic conditions, or methanolysis under anhydrous methanol-HCl conditions, and the stability of the glycosidic bond (the bonding between the reducing end glucose residue and the $-OR^2$ group) under acidic conditions was unknown, it was unknown whether the modified oligosaccharide derived from the oligosaccharide of the formula could withstand such reducing treatment. But unexpectedly, the present inventors found for the first time that ring-opening of the cyclic acetal portion was possible and accomplished the present invention.

The group of the formula: $-OR^2$ is bonded to the reducing end glucose residue and as mentioned above can be hydrolyzed by the action of glucoamylase- [E.C.3.2.1.3 ], α-glucosidase [E.C.3.2.1.20.], β-glucosidase [E.C.3.2.1.21.], isomaltase-[E.C.3.2.1.10] or β-amylase [E.C.3.2.1.2.], to form nitrophenols which have absorptions by themselves at visible light range, to finally form dyes by coupling with couplers by the action of oxidases such as catechol oxidase, laccase, tyrosinase and monophenol oxidase, or to finally form dyes by coupling with couplers by the action of oxidants.

Further examples of the group of the formula: $-OR^2$ are a 2-chloro-4-nitrophenoxy group a 4-trifluoromethylumbelliferyl group, a 5-bromoindoxyl group and a 4-chloro-3-bromoindoxyl group.

The modified oligosaccharide of this invention can effectively be used as a substrate for a fractional measuring method of α-amylase derived from the salivary glands and α-amylase derived from pancreas, that is, a fractional measuring method of α-amylase isozymes wherein decomposed products produced by hydrolysis of α-amylase are reacted with two or more coupling enzymes having different substrate specificities and the produced products are measured to conduct fractional measurements of α-amylase derived from human pancreas and α-amylase derived from human salivary glands.

This invention is illustrated by way of the following Examples.

PREPARATION EXAMPLE 1

Synthesis of mono-6-0-p-toluenesulfonyl β-cyclodextrin

In 100 ml of pyridine, 5.0 g of β-cyclodextrin was dissolved and 0.8 g of p-toluenesulfonyl chloride was added thereto, followed by stirring at 4 °C for 15 hours. After concentration under reduced pressure, the reaction solution was charged in a column (5 x 60 cm) packed with activated carbon and eluted with water, 30% ethanol, 25% n-propanol, each in amounts of 3 liters. The 25% n-propanol fractions were collected and concentrated under reduced pressure to yield 0.7 g of 6-O-p-toluene-sulfonyl β-cyclodextrin.

**Example 1**

Synthesis of p-nitrophenyl O-(6-O-benzyl)-α-D-glycopyranosyl-(1→4)-O-α-D-glucopyranosyl-(1→4)-O-α-D-glucopyranosyl-(1→4)-O-α-D-glucopyranosyl-(1→4)-α-D-glucopyranoside (hereinafter referred to as "BG5P")

(1) Synthesis of mono-o-benzyl-β-cyclodextrin (hereinafter referred to as "benzyl-β-CD")

In 120 ml of water, 15 g of β-cyclodextrin and 13.5 g of sodium hydroxide were dissolved, and 15 ml of benzyl chloride was added thereto. The reaction was carried out at 10 °C for 2 hours with stirring. After the reaction, the pH was made 7.0 with 6N HCl and 1 liter of acetone gas added thereto. The deposited precipitate gas filtered and 5 g of benzyl-β-CD was obtained (a mixture of substituted bodies having substituents at $C_2$, $C_3$, and $C_6$ positions).

(2) Synthesis of BG5P

In 1 liter of 10 mM ammonium acetate buffer (pH 6.5), 10 g of benzyl-β-CD and 10 g of p-nitrophenyl α-D-glucopyranoside were dissolved and 1000 kU of CGTase was added thereto. The reaction was carried out at 37 °C for 5 hours with stirring. After making the pH 4.0 with acetic acid, 20 kU of glucoamylase was added thereto and the reaction was carried out at 37 °C for 10 hours. After freeze-drying the reaction solution, purification was carried out by using a column (30 x 1500 mm) packed with Bio-Gel P-2 (mfd. by Bio-Rad Co.) equilibrated with 50 mM acetic acid to give 1.6 g of crude BG5P (a mixture of compounds having substituents at $C_2$, $C_3$, and $C_6$ positions). This was separated and purified by high performance liquid chromatography (HPLC) to give 180 mg of $C_6$ position substituted body (700 mg of $C_2$ position substituted body and 650 mg of $C_3$ position substituted body).

HPLC: content 96% [column: silica gel Z-ODS, $5C_{18}$ (a trade name, mfd. by Wako Pure Chemical Industries, Ltd., 10 x 300 mm); eluate: linear gradient of 10% $CH_3CN$ - 0.1% AcOH and 90% $CH_3CN$ - 0.1% AcOH, flow rate 3 ml/min, measured at 305 nm]

IR: as shown in Fig. 1.

$^1$H-NMR: as shown in Fig. 2.

G L.C. analyses ... Glucose residue and benzyl glucose residue in BG5P were analyzed by G.L.C. (column, 2% OV-17 on chromosorb mfd. by Wako Pure Chem. Ind. Ltd., 0.4 x 200 cm) after methanolysis (1.4 M HCl-methanol, 90 °C for 2h) followed by trimethylsilylation with hexamethyldisilazane and trimethylsilyl chloride in pyridine. The temperature was programmed from 110 to 250 °C at the rate of 4 °C increments per minute. The concentration of p-nitrophenyl group was taken as unity, and the content was estimated from the absorbance at 305 nm in 0.1 M acetic acid solution.

From the results mentioned above and the fact that BG5P was not acted by glucoamylase, the structure of BG5P was estimated as follows:

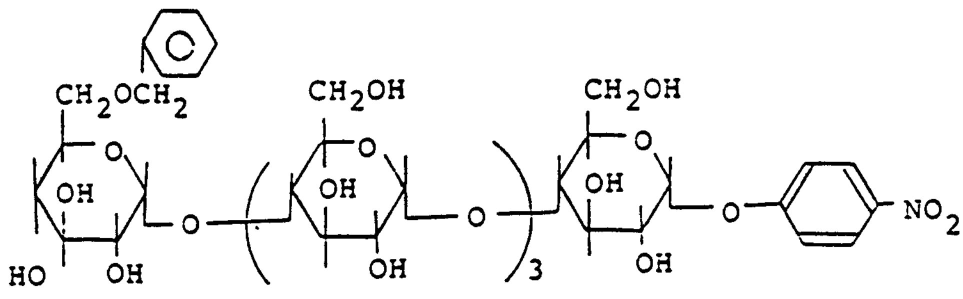

Example 2

Synthesis of p-nitrophenyl O-(6-bromo-6-deoxy)-α-D-glycopyranosyl-(1→4)-O-α-D-glucopyranosyl-(1→4)-O-α-D-glucopyranosyl-(1→4)-α-D-glucopyranoside (hereinafter referred to as "BrG5P")

(1) Synthesis of mono-6-bromo-6-deoxy-β-cyclodextrin (hereinafter referred to as "Br-β-CD")

In 250 ml of DMF, 5 g of mono-6-O-p-toluene-sulfonyl β-cyclodextrin synthesized according to Preparation Example 1 was dissolved and 15 g of lithium bromide was added thereto. The reaction was carried out for 2 hours with refluxing. After concentrating under reduced pressure, 500 ml of acetone was added thereto to deposite a precipitate, which was filtered to give 2.3 g of mono-6-bromo-6-deoxy-β-cyclodextrin.

(2) Synthesis of BrG5P

In 100 ml of 10 mM ammonium acetate buffer (pH 6.5), 1 g of Br-β-CD and 1 g of p-nitrophenyl α-D-glucopyranoside were dissolved and 100 kU of CGTase was added thereto. The reaction was carried out at 37°C for 5 hours with stirring. After making the pH 4.0 with acetic acid, 2000 U of glucoamylase was added thereto and the reaction was carried out at 37°C for 10 hours with stirring. The reaction solution was concentrated and purified by a column (30 x 1500 mm) packed with Bio-Gel P-2 (mfd. by Bio-Rad Co.) equilibrated with 50mM acetic acid to give 0.16 g of BrG5P.

HPLC: content 97% (measuring conditions were the same as described in Example 1)

$^1$H-NMR: as shown in Fig. 3.

The ratio of glucose/p-nitrophenol of BrG5P obtained in the same manner as described in Example 1 was 3.6.

From the results mentioned above and the fact that BrG5P was not acted by glucoamylase, the structure of BrG5P was estimated as follows:

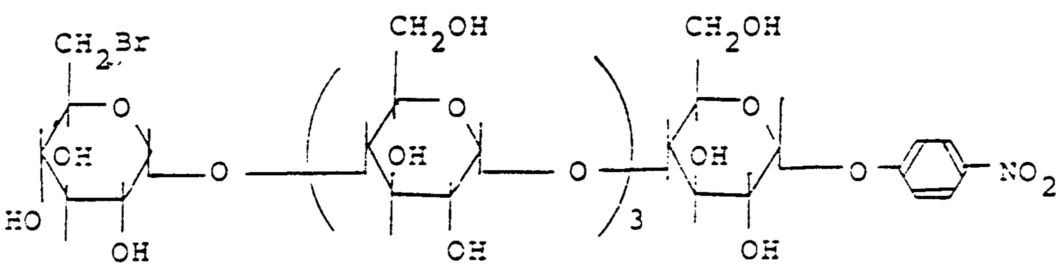

Example 3

Synthesis of BG5P

After dissolving 3 g of p-nitrophenyl-α-D-maltopentaoside in 50 ml of DMF, 15 ml of benzaldehyde dimethylacetal and 1 g of p-toluenesulfonic acid (anhydride) were added thereto. The reaction was carried out at 50°C for 3 hours with stirring. After the reaction, 100 ml of pyridine was added thereto, and 60 ml of

benzoyl chloride was added dropwise to carry out the reaction. The reaction solution was poured into a solution of saturated sodium hydrogen carbonate to stop the reaction. The reaction solution was extracted with 200 ml of chloroform and washed with saturated saline solution twice, and the solvent was removed in vacuo. The resulting syrup was transferred to a 1-liter four-necked flask and dissolved in 250 ml of THF, followed by addition and dissolution of 3 g of dimethylamine borane. To this solution, 10 ml of a solution of 0.5 M HCl (gas) in diethyl ether was added dropwise with stirring, and the reaction solution was poured into 200 ml of saturated sodium hydrogen carbonate to stop the reaction, followed by extraction with 200 ml of chloroform. After washing twice with saturated saline solution, the solvent was removed in vacuo. To the resulting solution, 500 ml of methanol solution containing 0.1N sodium methoxide was added and stirred at 25 °C for 4 hours, followed by addition of acetic acid for neutralization. After removing the solvent in vacuo, 20 ml of 50 mM acetic acid solution was added and applied to a Bio-Gel p-2 column (mfd. by Bio-Rad Co., 2 cm in diameter x 150 cm) equilibrated with 50 mM acetic acid and the BG5P fractions were collected and freeze-dried.

Yield: 450 mg

IR chart: as shown in Fig. 1

$^1$H-NMR: as shown in Fig. 2

HPLC: content 95% (measuring conditions were the same as described in Example 1)

GC composition analysis: glucose/6-O-benzyl-glucose/p-nitrophenyl group = 3.8/0.9/1.0 (measuring conditions were the same as described in Example 1)

Example 4

Measurement of α-amylase activity

[Measuring reagent]

A measuring reagent solution was prepared by dissolving 30 mg of BG5P obtained in Example 1 in 30 ml of 50 mmol/l 2-(N-morpholino)ethanesulfonic acid (MES)-NaOH buffer (pH 6.9) containing 400 units of glucoamylase, 300 units of α-glucosidase and 20 mmol/l of calcium chloride.

[Measuring procedure]

To 2 ml of the measuring solution, 100 µl of a sample serum was added and incubated at 37 °C. The change of absorbance of the reaction solution at a wavelength of 405 nm was measured.

On the other hand, using a standard sample containing known activities of α-amylase, the calibration curve was prepared in the same manner as mentioned above. From this calibration curve, α-amylase activity of a sample serum was obtained. Fig. 4 shows a relationship between the α-amylase activity at individual diluted stages of a standard sample (Somogyi unit/dl) and the increase in absorbance (ΔA) per minute at a wavelength of 405 nm.

As is clear from Fig. 4 the calibration curve obtained by lining the plots of increased amounts of absorbance (ΔA/min) corresponding to α-amylase activity (Somogyi unit/dl) is linear and passing through the zero point. This means that the calibration curve shows good quantitativeness.

Example 5

Measurement of α-amylase activity

[Measuring reagent]

A measuring reagent solution was prepared by dissolving 2.1 g of N,N-bis(2-hydroxyethyl)-2-amino-ethanesulfonic acid, 230 mg of NaCl, 58 mg of $CaCl_2$, 500 mg of NaOH in distilled water, making the total amount 100 ml (pH 7. 3) and dissolving 125 mg of BG5P obtained in Example 3 therein.

Solution Ⓐ : To 50 ml of the resulting solution, 25,000 units of α-glucosidase was added.

Solution Ⓑ : To 20 ml of the solution Ⓐ , 1000 units of glucoamylase was added.

[Measuring procedure]

To 2 ml of the reagent solution Ⓐ or Ⓑ , 50 µl of serum sample was added and incubated at 37 °C. The change of absorbance of the reaction solution at a wavelength of 405 nm was measured.

Increasing amounts of absorbances (ΔA) per minute when measured by using 5 kinds of human serum at a wavelength of 405 nm were shown in Table 4.

Table 4

| Human serum No. | Reagent solution | |
|---|---|---|
| | Ⓐ | Ⓑ |
| 1 | 0.0330 | 0.0335 |
| 2 | 0.0291 | 0.0291 |
| 3 | 0.0329 | 0.0330 |
| 4 | 0.0533 | 0.0537 |
| 5 | 0.0886 | 0.0885 |

As is clear from Table 4, the measuring sensitivity is hardly influenced by the addition of glucoamylase.

Example 6

Measurement of α-amylase activity

[Measureing reagent]

A measuring reagent solution was prepared by dissolving 30 mg of BrG5P obtained in Example 2 in 30 ml of 50 mmol/l MES-NaOH buffer (pH 6.9) containing 400 units of glucoamylase, 300 units of α-glucosidase and 20 mmol/l of calcium chloride.

[Measuring procedure]

To 2 ml of the measuring solution, 100 µl of a serum sample was added and incubated at 37 °C. The change of absorbance of the reaction solution at a wavelength of 405 nm was measured.

On the other hand, using a standard sample containing known activities of α-amylase, the calibration curve was prepared in the same manner as mentioned above. From this calibration curve, α-amylase activity was obtained. Fig. 5 shows a relationship between the α-amylase activity at individual diluted stages of a standard sample (Somogyi unit/dl) and the increase in absorbance (ΔA) per minute at a wavelength of 405 nm.

As is clear from Fig. 5 , the calibration curve obtained by lining the plots of increased amounts of absorbance (ΔA/min) corresponding to α-amylase activity (Somogyi unit/dl) is linear and passing through the zero point. This means that the calibration curve shows good quantitativeness.

## Claims

**1.** A process for determining the activity of α-amylase in a sample which comprises
using as a substrate for α-amylase a modified oligosaccharide of the formula:

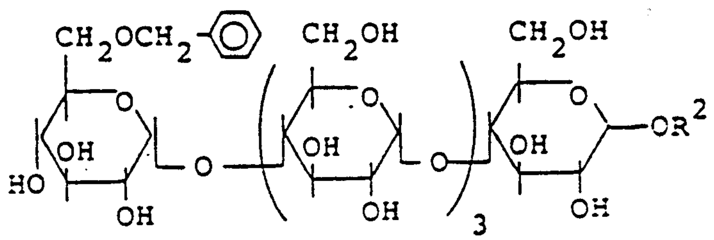

(I)

or

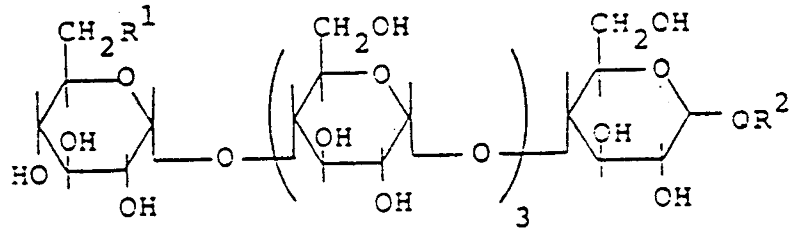

(II)

wherein $R^1$ is a halogen atom; and $R^2$ is a group of the formula:

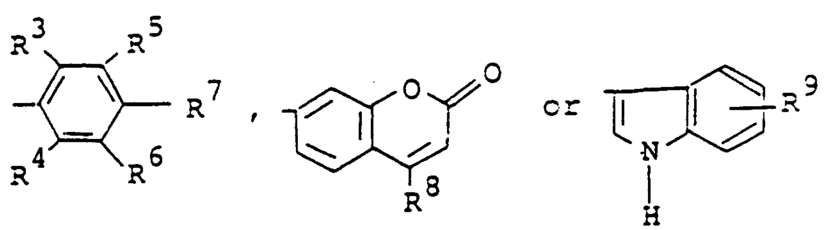

in which $R^3$ through $R^6$ are independently hydrogen, a lower alkyl group, a lower alkoxy group, a nitro group, a carboxyl group, a sulfonic acid group or a halogen atom, and $R^3$ and $R^5$, and/or $R^4$ and $R^6$, may be bonded to form an aromatic ring; $R^7$ is hydrogen, a lower alkoxy group, a halogen atom, or a nitro group; $R^8$ is hydrogen, a methyl group, or a trifluoromethyl group; and $R^9$ is hydrogen or a halogen atom,

contacting said sample with said substrate in the presence of at least one exo-enzyme, and measuring an optically measurable change as a measure of $\alpha$-amylase activity in said sample.

2. A process according to Claim 1, wherein the modified oligasoccharide is represented by the formula:

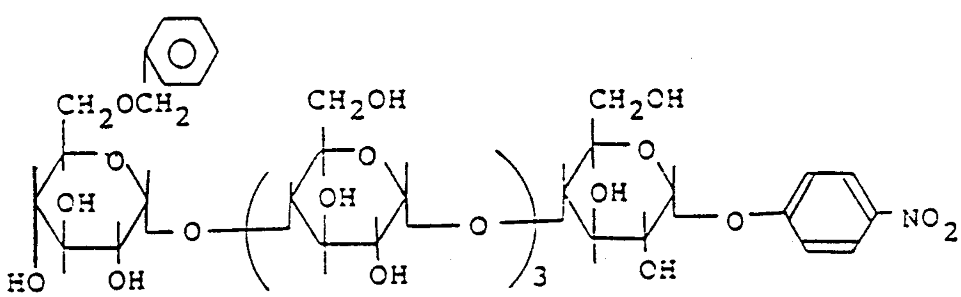

3. A process according to Claim 1, wherein the modified oligosaccharide is represented by the formula:

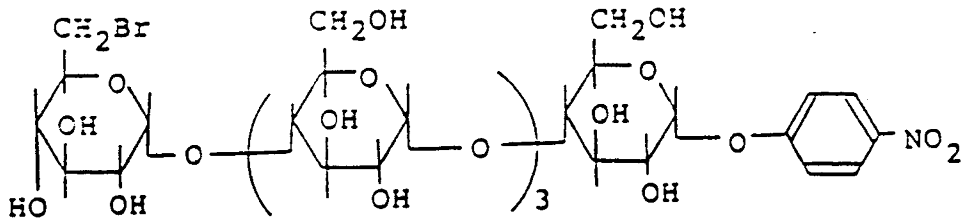

4. A process according to Claim 1, wherein the modified oligosaccharide of the formula (I) or (II) is produced by reacting a modified cyclodextrin with cyclomaltodextrin-glucanotransferase in the presence of an acceptor selected from glucose, maltose, and maltotriose wherein said glucose, maltose and maltotriose each carry a substituent linked to the sugar moiety by a glycosidic bond which upon cleavage exhibits an optically measurable change, and then reacting with glucoamylase or α-glucosidase.

5. A process according to claim 1, wherein the modified oligosaccharide of the formula (I) is produced by reacting an oligosaccharide having a substituent which exhibits an optically measurable change upon cleavage at the reducing-end glucose residue with either benzaldehyde or benzaldehyde dimethylacetal to form the 4,6-0-cyclic acetal at the non-reducing-end glucose residue, and reducing said 4,6-0-cyclic acetal.

## Patentansprüche

1. Verfahren zur Bestimmung der Aktivität von alpha-Amylase in einer Probe, welches umfaßt:

Verwenden eines modifizierten Oligosaccharids als ein Substrat für alpha-Amylase, welches Oligosaccharid die Formel hat:

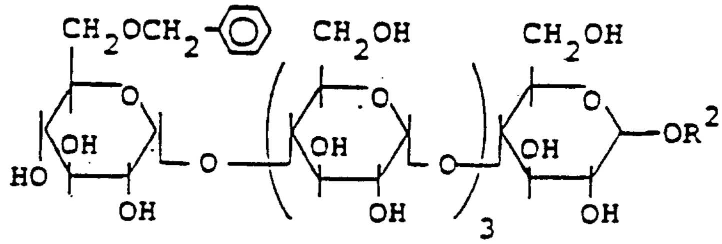

(I)

oder

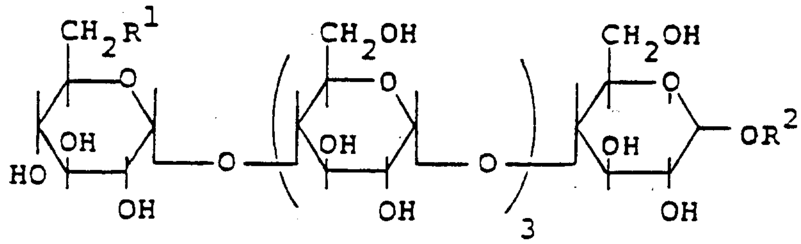

(II)

worin sind: $R^1$ ein Halogenatom und $R^2$ eine Gruppe der Formel:

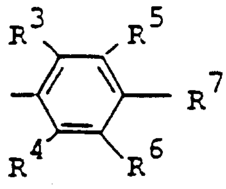 , 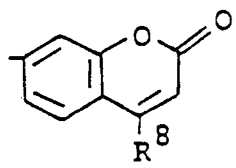 oder 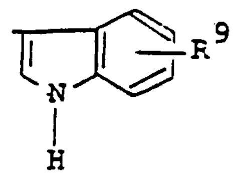

worin $R^3$ bis $R^6$ voneinander unabhängig Wasserstoff, eine niedere Alkylgruppe, eine niedere Alkoxygruppe, eine Nitrogruppe, eine Carboxylgruppe, eine Sulfonsäuregruppe oder ein Halogenatom sind und $R^3$ und $R^5$ und/oder $R^4$ und $R^6$ zur Bildung eines aromatischen Ringes gebunden sein können; $R^7$ Wasserstoff, eine niedere Alkoxygruppe, ein Halogenatom oder eine Nitrogruppe ist; $R^8$ Wasserstoff, eine Methylgruppe oder eine Trifluormethylgruppe ist und $R^9$ Wasserstoff oder ein Halogenatom ist,

Inkontaktbringen der Probe mit dem Substrat in Gegenwart mindestens eines Exoenzyms und

Messen einer optisch meßbaren Änderung als Maß der alpha-Amylaseaktivität in der Probe.

**2.** Verfahren nach Anspruch 1, bei welchem das modifizierte Oligosaccharid dargestellt wird durch die Formel:

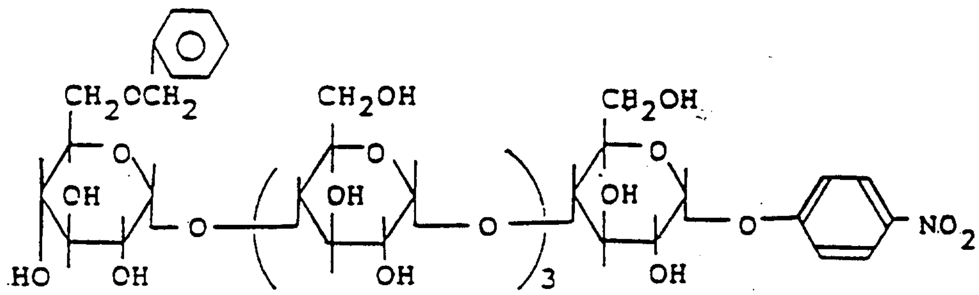

**3.** Verfahren nach Anspruch 1, bei welchem das modifizierte Oligosaccharid dargestellt wird durch die Formel:

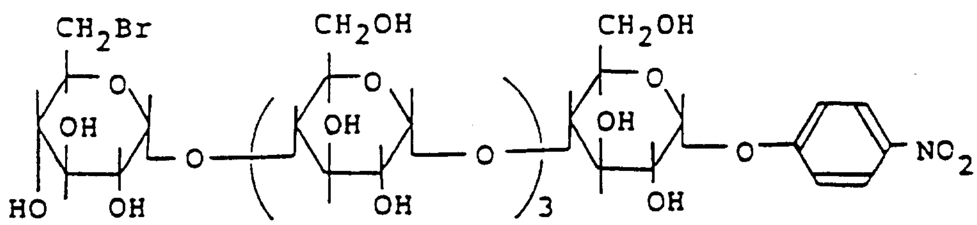

**4.** Verfahren nach Anspruch 1, bei welchem das modifizierte Oligosaccharid der Formel (I) oder (II) hergestellt wird durch Umsetzen eines modifizierten Cyclodextrins mit Cyclomaltodextrin-glucanotransferase in Gegenwart eines Akzeptors, der ausgewählt wird aus Glucose, Maltose und Maltotriose, worin die Glucose, Maltose und Maltotriose jede einen Substituenten aufweisen, der an dem Zucker-Bruchstück über eine glykosidische Bindung verknüpft ist, das bei der Aufspaltung eine optisch meßbare Änderung zeigt, und sodann Umsetzen mit Glucoamylase oder alpha-Glucosidase.

**5.** Verfahren nach Anspruch 1, bei welchem das modifizierte Oligosaccharid der Formel (I) hergestellt wird durch Umsetzen eines Oligosaccharids, das einen Substituenten aufweist, der eine optisch meßbare Änderung bei Aufspaltung an dem reduzierenden Ende des Glucoserests zeigt, mit entweder Benzaldehyd oder Bendaldehyddimethylacetal, um das 4,6-O-cyclische Acetal an dem nichtreduzierenden Ende des Glucoserests zu binden und Reduzieren des 4,6-O-cyclischen Acetals.

## Revendications

**1.** Procédé de détermination de l'activité d'une α-amylase dans un échantillon, qui comprend l'utilisation comme substrat pour l'α-amylase d'un oligosaccharide de formule :

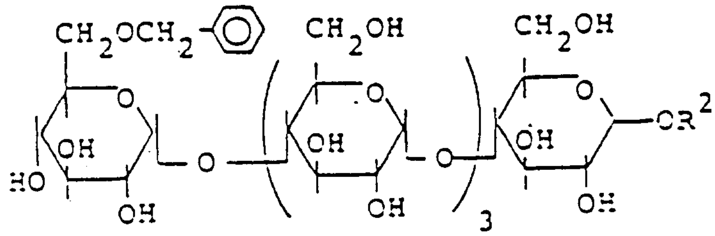

(I)

ou

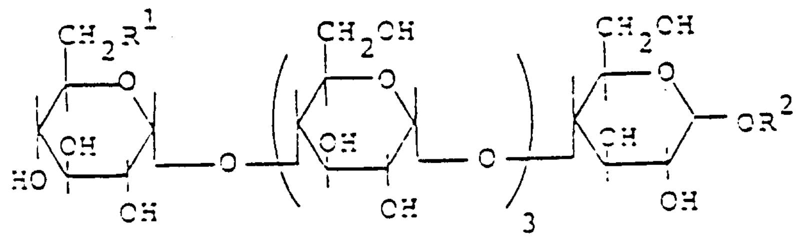

(II)

où $R^1$ est un atome d'halogène ; et $R^2$ est un groupe de formule :

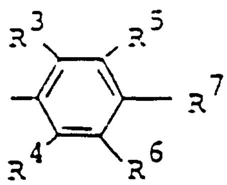

,

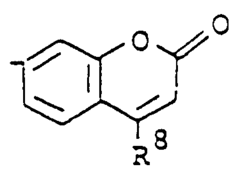

ou

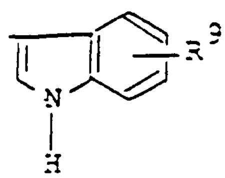

dans lequel $R^3$ à $R^6$ sont indépendamment de l'hydrogène, un groupe alkyle inférieur, un groupe alkoxy inférieur, un groupe azoté, un groupe carboxyle, un groupe d'acide sulfonique ou un atome d'halogène, et $R^3$ et $R^5$, et/ou $R^4$ et $R^6$, peuvent être liés pour former un cycle aromatique ; $R^7$ est de l'hydrogène, un groupe alkoxy inférieur, un atome d'halogène, ou un groupe azoté ; $R^8$ est de l'hydrogène, un groupe méthyle, ou un groupe trifluorométhyle ; et $R^9$ est de l'hydrogène ou un atome d'halogène,

la mise en contact dudit échantillon avec ledit substrat en présence d'au moins une exo-enzyme, et

la mesure d'une modification optiquement mesurable comme mesure de l'activité de l'α-amylase dans ledit échantillon.

**2.** Procédé selon la revendication 1, dans lequel l'oligosaccharide modifié est représenté par la formule :

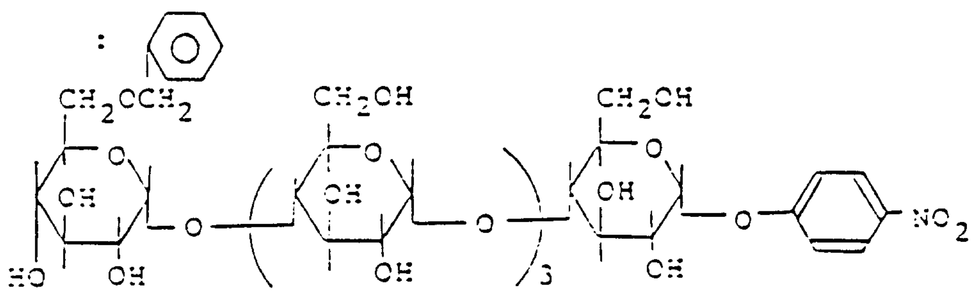

3. Procédé selon la revendication 1, dans lequel l'oligosaccharide modifié est représenté par la formule :

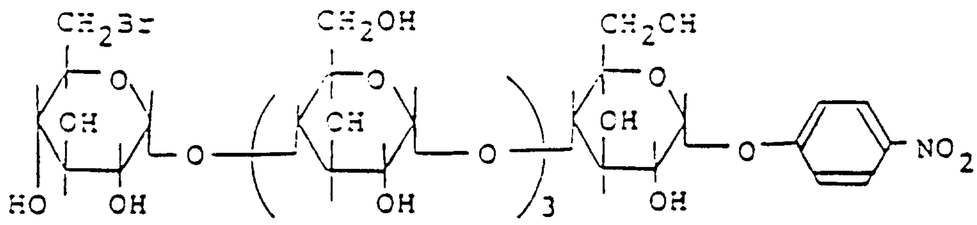

4. Procédé selon la revendication 1, dans lequel l'oligosaccharide modifié de formule (I) ou (II) est produit en faisant réagir une cyclodextrine modifiée avec une cyclomaltodextrine-glucanotransférase en présence d'un accepteur choisi parmi le glucose, le maltose, et la maltotriose, lesdits glucose, maltose et maltotriose portant chacun un substituant lié à la partie de sucre par une liaison glucosidique qui, lors de la coupure, présente une modification optiquement mesurable, puis en faisant réagir avec de la glucoamylase ou de l'α-glucosidase.

5. Procédé selon la revendication 1, dans lequel l'oligosaccharide modifié de formule (I) est produit en faisant réagir un oligosaccharide portant un substituant qui présente une modification optiquement mesurable lors de la coupure au niveau du résidu de glucose à extrémité réductrice, avec du benzaldéhyde ou du diméthylacétal de benzaldéhyde, pour former l'acétal 4,6-O-cyclique au niveau du résidu de glucose à extrémité non réductrice, et en réduisant ledit acétal 4,6-O-cyclique.

FIG. 1

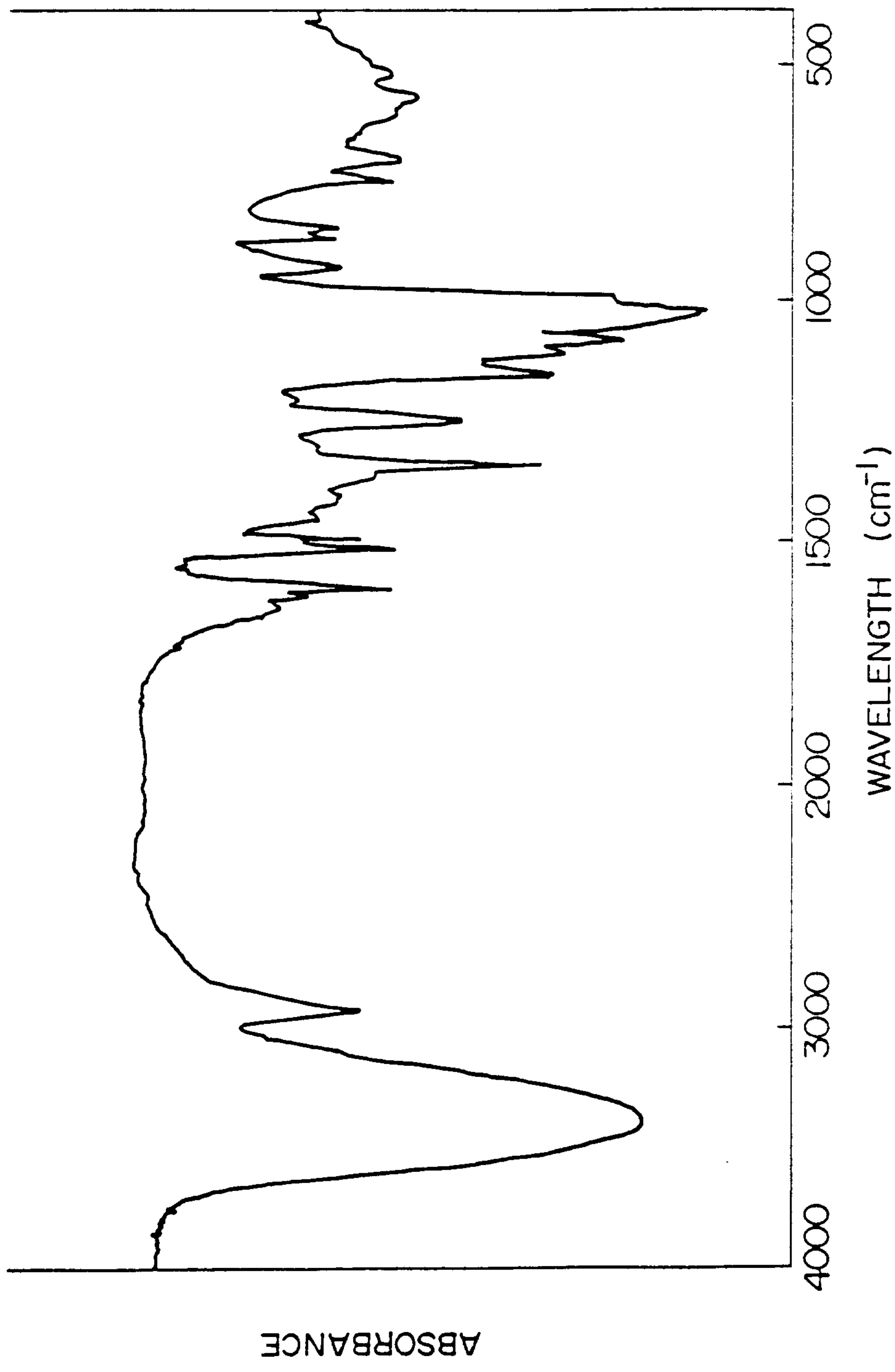

FIG. 2

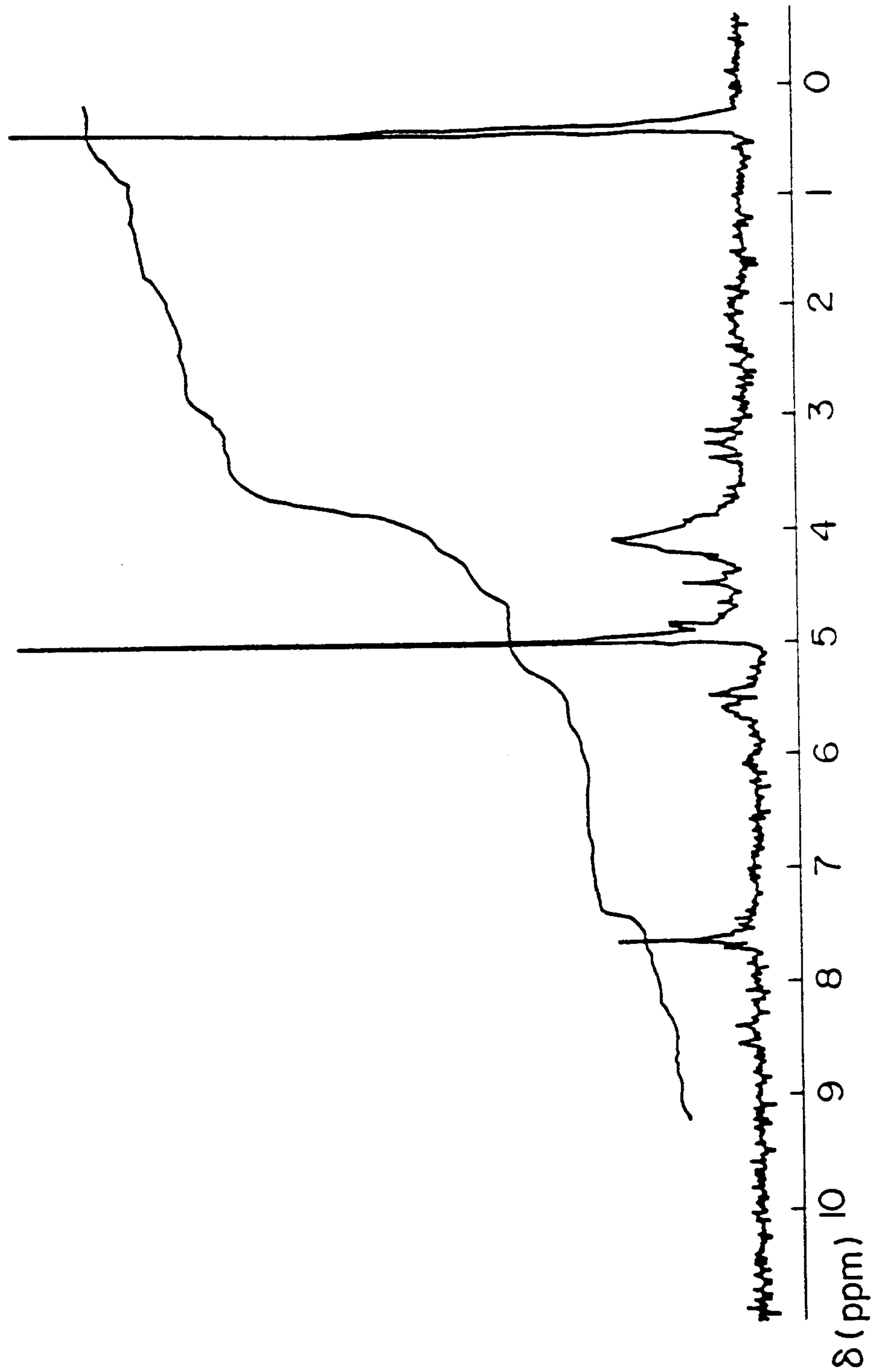

FIG. 3

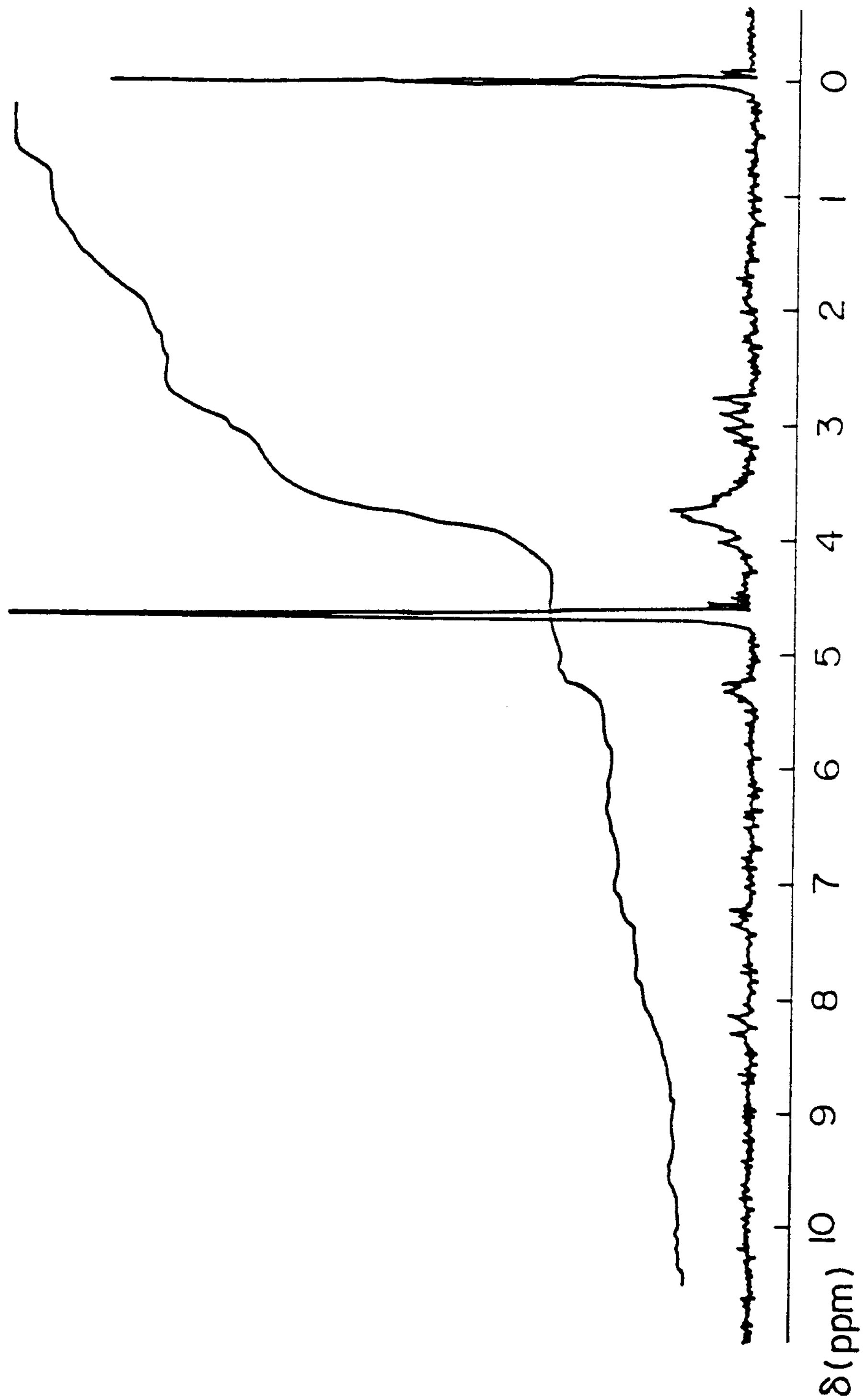

FIG. 4

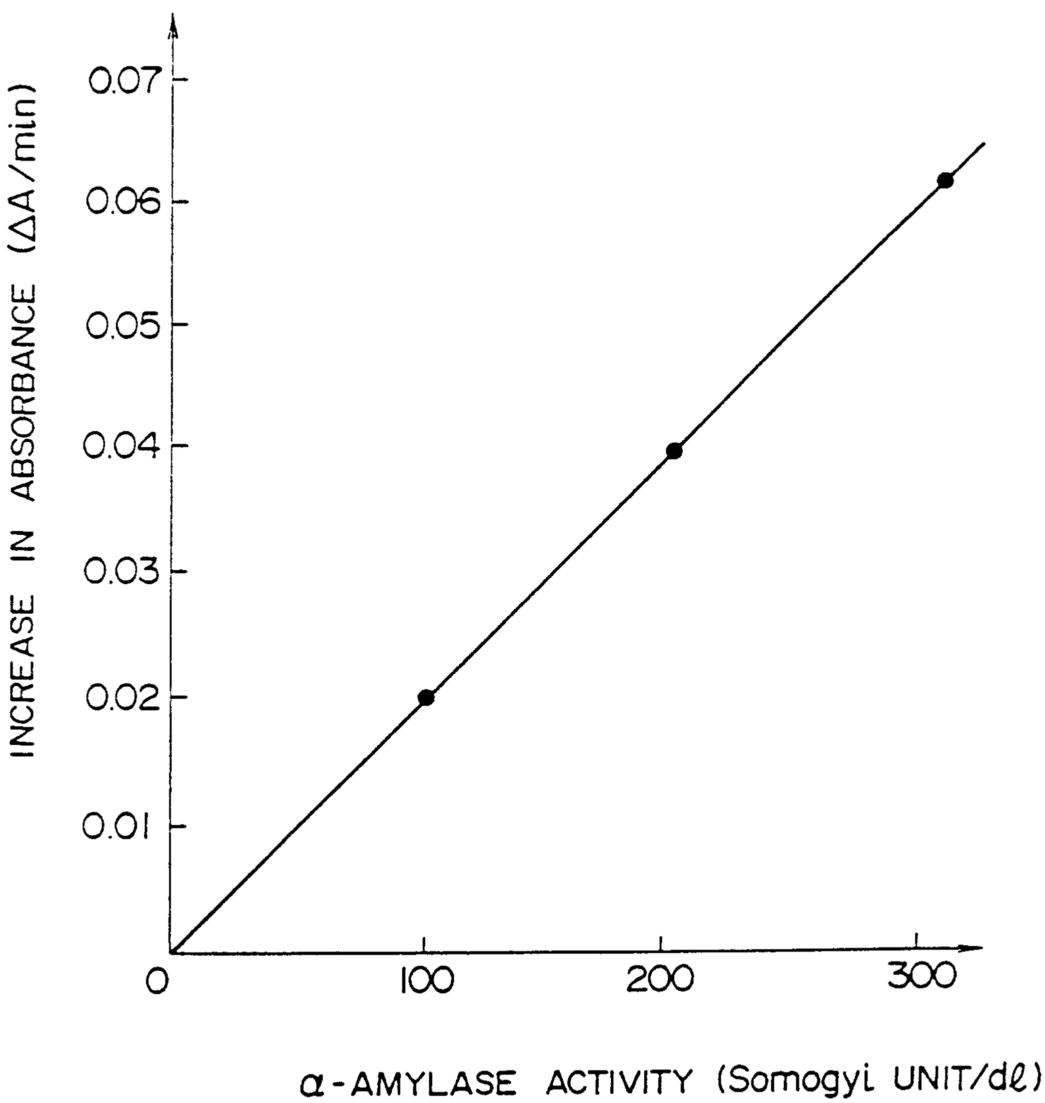
INCREASE IN ABSORBANCE (ΔA/min)
0.07
0.06
0.05
0.04
0.03
0.02
0.01
0
100
200
300
α-AMYLASE ACTIVITY (Somogyi UNIT/dℓ)

FIG. 5

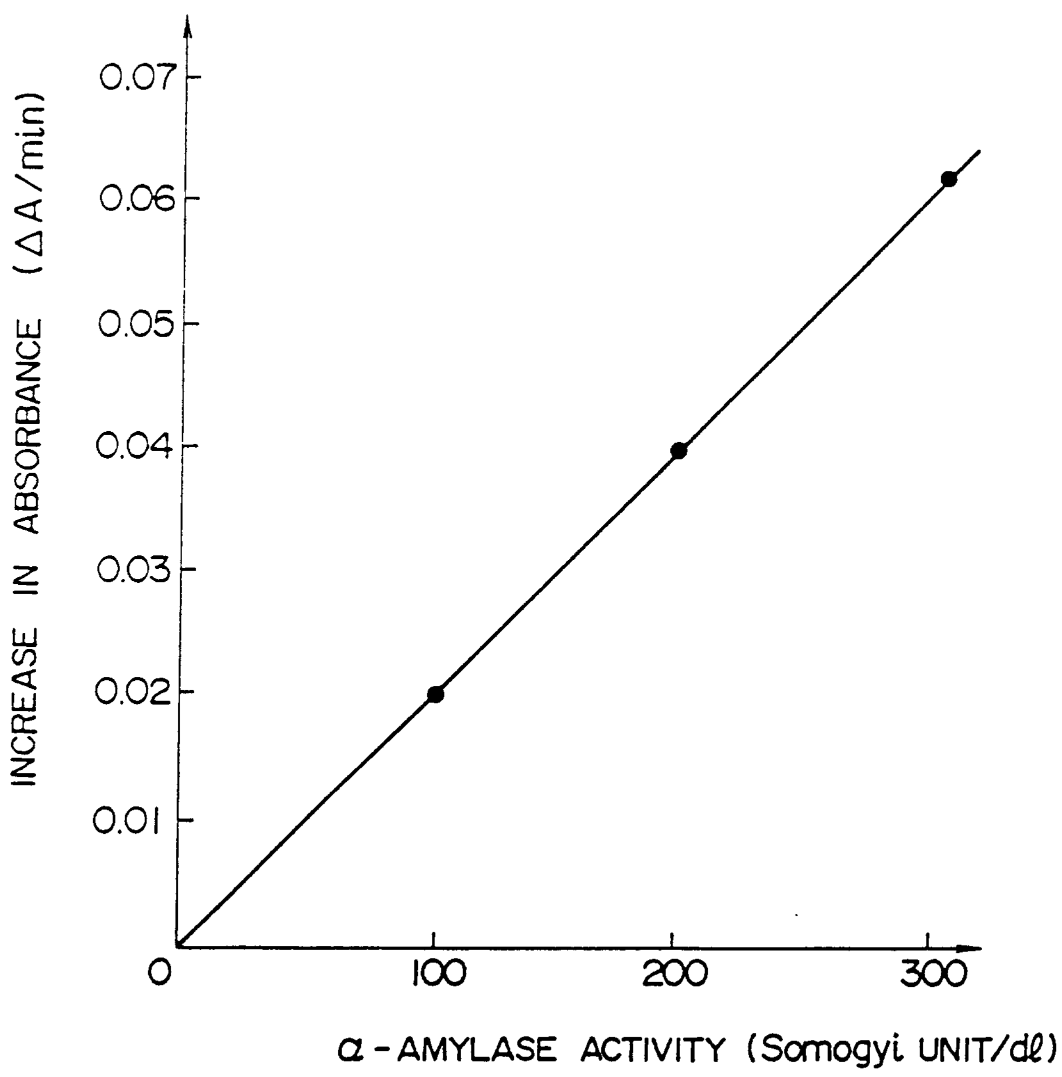
INCREASE IN ABSORBANCE (ΔA/min)
0.07
0.06
0.05
0.04
0.03
0.02
0.01
0
100
200
300
α-AMYLASE ACTIVITY (Somogyi UNIT/dℓ)